# EUROPEAN PATENT APPLICATION

(11) **EP 4 269 597 A1**
(43) Date of publication of application: **01.11.2023**
(21) Application number: 21911588.8
(22) Date of filing: 23.12.2021
(51) Int. Cl.: C12N 15/77, C12N 15/70, C07K 14/195, C12P 13/24

(54) **L-HISTIDINE EXPORT PROTEIN AND METHOD OF PRODUCING L-HISTIDINE USING SAME**

(30) Priority: 24.12.2020 KR 20200183702
(71) Applicant: CJ Cheiljedang Corporation, Seoul 04560 (KR)
(72) Inventor: HUH, Lan, Seoul 04560 (KR); SEO, Chang Il, Seoul 04560 (KR); CHEONG, Ki Yong, Seoul 04560 (KR); LIM, Su-Bin, Seoul 04560 (KR)
(74) Representative: Bates, Philip Ian
(86) International application number: PCT/KR2021/019769
(87) International publication number: WO 2022/139523

(57) **Abstract**

Provided are a novel protein having histidine exporting activity, an L-histidine producing microorganism modified to express the protein, and a L-histidine producing method using the microorganism.

## Description

### [TECHNICAL FIELD]

Related are a novel protein having histidine export activity, an L-histidine producing microorganism modified to express the protein, and a method for producing L-histidine using the microorganism.

### [BACKGROUND ART]

L-histidine is one amino acid of 20 standard amino acids, and from a nutritional point of view, it is not required in a large amount for adults, but it is classified as an essential amino acid for growing children. In addition, L-histidine is involved in important physiological processes such as anti-oxidation and immune regulation and the like, and is used in the medical industry such as a raw material of gastric ulcer therapeutic agent or a circulatory system therapeutic agent, and an amino acid infusion preparation and the like.

Since L-histidine is particularly abundant in hemoglobin, it is mainly produced through a proteolytic extraction method using blood meal as a raw material. However, this method has disadvantages such as low efficiency and environmental pollution, and the like. On the other hand, it is possible to produce L-histidine through a microbial fermentation method, but large-scale industrialization has not yet been achieved. This is because biosynthesis of L-histidine competes with phosphoribosyl pyrophosphate (PRPP), a nucleotide synthesis precursor, and has a complex biosynthesis process and a regulatory mechanism that require high energy.

An example that production of the corresponding amino acid increases when expression and/or function of a protein having export ability of other kinds of amino acids has been known, but almost no previous studies on a protein having export ability specific to L-histidine have been conducted.

Under this background, the discovery of a protein having export ability specific to histidine and development of a histidine producing technology using this are required.

### [DISCLOSURE]

### [TECHNICAL PROBLEM]

In the present description, it is proposed that the L-histidine production can be remarkably improved as a result of discovering a histidine export protein having L-histidine export ability, and expressing this in a microorganism having producing ability of L-histidine.

One embodiment provides a protein having L-histidine export activity. The protein may be a protein having export ability specific to L-histidine.

Another embodiment provides an L-histidine producing microorganism, which expresses the L-histidine export protein.

Other embodiment provides a method for producing L-histidine, comprising culturing the microorganism in a medium.

### [TECHNICAL SOLUTION]

In the present description, by discovering a histidine export protein having L-histidine export ability, and introducing this into a microorganism having producing ability of L-histidine, a recombinant microorganism with dramatically improved L-histidine production and a technology for producing L-histinde using this are provided.

Hereinafter, they will be described in more detail.

One embodiment provides a protein having L-histidine export activity. The protein may be a protein having export ability specific to L-histidine. In the present description, the protein may be represented by an L-histidine export protein. In one embodiment, the L-histidine export protein may have L-histidine export ability in a microorganim of the genus *Corynebacterium* and/or the genus *Escherichia,* and then, the L-histidine export protein may be a protein derived from a microorganism which does not belong to the genus *Corynebacterium* and/or the genus *Escherichia,* for example, at least one microorganism selected from the group consisting of the genus *Dermabacter* (e.g., *Dermabacter vaginalis,* etc.), the genus *Helcobacillus* (e.g., *Helcobacillus massiliensis*, etc.), the genus *Mycobacterium* (e.g., *Mycobacterium abscessus subsp. abscessus*, etc.), and the like.

In one embodiment, the L-histidine export protein may be a protein having at least 60% sequence homology with SEQ ID NO: 12, SEQ ID NO: 13 or a combination thereof. For example, in one specific embodiment, the L-histidine export protein may have at least 60%, 65%, 70%, 75%, 80%, 85%, 90%, 91%, 92%, 93%, 94%, 95%, 96%, 97%, 98%, 99%, or 99.5% homology with SEQ ID NO: 12, 13, or a combination thereof.

In one specific embodiment, the L-histidine export protein may be at least one kind, for example, one kind, 2 kinds or 3 kinds selected from the group consisting of proteins which comprise an amino acid sequence selected from the following or consist of the sequence:
SEQ ID NO: 12, SEQ ID NO: 13, or a combination thereof;
SEQ ID NO: 41, SEQ ID NO: 42, or a combination thereof; and
SEQ ID NO: 44, SEQ ID NO: 45, or a combination thereof.

The protein represented by SEQ ID NO: 12 may be encoded by the nucleic acid sequence of SEQ ID NO: 64, and the protein represented by , SEQ ID NO: 13 may be encodied by the nucleic acid sequence of SEQ ID NO: 65, or the protein represented by SEQ ID NO: 12 and/or SEQ ID NO: 13 may be encoded by the nucleic acid sequence of SEQ ID NO: 14 (an operon sequence fused at the overlapping region of the 3' end of SEQ ID NO: 64 and the 5' end of SEQ ID NO: 65).

The protein represented by SEQ ID NO: 41 may be encoded by the nucleic acid sequence of SEQ ID NO: 66, and the protein represented by , SEQ ID NO: 42 may be encodied by the nucleic acid sequence of SEQ ID NO: 67, or the protein represented by SEQ ID NO: 41 and/or SEQ ID NO: 42 may be encoded by the nucleic acid sequence of SEQ ID NO: 43 (an operon sequence fused at the overlapping region of the 3' end of SEQ ID NO: 66 and the 5' end of SEQ ID NO: 67).

The protein represented by SEQ ID NO: 44 may be encoded by the nucleic acid sequence of SEQ ID NO: 68, and the protein represented by , SEQ ID NO: 45 may be encodied by the nucleic acid sequence of SEQ ID NO: 69, or the protein represented by SEQ ID NO: 44 and/or SEQ ID NO: 45 may be encoded by the nucleic acid sequence of SEQ ID NO: 46 (an operon sequence fused at the overlapping region of the 3' end of SEQ ID NO: 68 and the 5' end of SEQ ID NO: 69).

Another embodiment provides an L-histidine producing microorganism, modified to express an L-histidine export protein. The L-histidine export protein is as described in advance. The L-histidine export protein may be a protein derived from foreign proteins for the L-histidine producing microorganism, for example, heterogenous microorganisms from the microorganism.

In the present description, the term "L-histidine producing microorganism"
may be used to mean
a case of having increased L-histidine producing ability compared to a non-modified microorganism, by modifying a microorganism having L-histidine producing ability to express the L-histidine export protein, for example, by modifying it 1) to further express the L-histidine export protein, or 2) to express it by replacing an endogenous L-histidine export protein, and/or
a case of having L-amino acid producing ability by modifying a microorganism having no L-histidine producing ability to express the L-histidine export protein.

In the present description, "microorganism" encompasses single-celled bacteria, and may be used interchangeably with "cell."

In the present description, the non-modified microorganism is used to distinguish it from "L-histidine producing microorganism" which is modified to express an L-histidine export protein, so the L-histidine producing ability is increased or the L-hisitidine producing ability is given, and may mean a microorganism before being modified to express the L-histidine export protein or a microorganism which is not modified to express the L-histidine export protein, and may be represented by a host microorganism.

The microorganism may be at least one selected from the group consisting of microorganisms naturally having L-histidine producing ability or all gram-positive bacterial which has no L-histidine producing ability or can have L-histidine producing ability by introducing a mutation into a significantly fewer strains, for example, microorganisms of the genus *Corynebacterium* and microorganisms of the genus *Escherichia.* The microorganism of the genus *Corynebacterium* may include *Corynebacterium glutamicum, Corynebacterium ammoniagenes, Brevibacterium lactofermentum*, *Brevibacterium flavum*, *Corynebacterium thermoaminogenes, Corynebacterium efficiens*, and the like, but not limited thereto. For example, the microorganism of the genus *Corynebacterium* may be *Corynebacterium glutamicum.*

In one embodiment, the L-histidine producing microorganism modified to express the L-histidine export protein may have increased L-histidine producing ability, compared to a homogeneous non-modified microorganism which is not modified to express the L-histidine export protein, for example, a foreign L-histidine export protein. In one specific embodiment, the L-histidine producing microorganism modified to express the L-histidine export protein may have L-histidine production (for example, content in a medium) by at least 5%(w/v), at least 10%(w/v), at least 12.5%(w/v), at least 15%(w/v), at least 17.5%(w/v), or at least 20%(w/v) (the upper limit of the L-histidine production increase rate may be not limited thereto, but may be 100%(w/v), 90%(w/v), 80%(w/v), 75%(w/v), 70%(w/v), 65%(w/v), 60%(w/v), 55%(w/v), or 50%(w/v)). Comparison of the L-histidine production between the L-histidine producing microorganism modified to express the L-histidine export protein and non-modified microorganism, may be performed based on the case where a substrate (for example, sugar such as glucose, etc.) is used in the same amount each other, and for example, it may be comparison of the L-histidine content in the substrate (for example, sugar such as glucose, etc.) unit amount (1g, 10g, or 100g, etc.) standard medium.

In the present description, the term "modified to express an L-histidine export protein" may mean any manipulation to express a foreign L-histidine export protein in a microorganism, and for example, it may mean introduction of a gene encoding a forcing L-histidine export protein in a microorganism or transformation of the microorganism with a gene encoding a foreign L-histidine export protein.

In the present description, that a polynucleotide (may be used with "gene" interchangeably) or a polypeptide (may be used with "protein" interchangeably) "comprises a specific nucleic acid sequence or an amino acid sequence, consists of a specific nucleic acid sequence or an amino acid sequence, or is represented by a specific nucleic acid sequence or an amino acid sequence" is an expression that can be used interchangeably as an equivalent meaning, and may mean that the polynucleotide or polypeptide consists of the specific nucleic acid sequence or amino acid sequence by essentially comprising it, and it may be interpreted as comprising a "substantially equivalent sequence" in which a mutation (deletion, substitution, modification and/or addition) is added to the specific nucleic acid sequence or amino acid sequence within a range of maintaining the original function and/or desired function of the polynucleotide or polypeptide (or not excluding that the mutation is introduced).

In one embodiment, the nucleic acid sequence or amino acid sequence provided in the present description may comprise that modified by common mutagenesis, for example, direct evolution and/or site-directed mutagenesis, and the like within a range of maintaining the original function or desired function thereof. In one embodiment, that a polynucleotide or a polypeptide "comprises a specific nucleic acid sequence or an amino acid sequence" may mean that the polynucleotide or polypeptide (i) consists of the specific nucleic acid sequence or amino acid sequence or essentially comprises it, or (ii) consists of a nucleic acid sequence or amino acid sequence having homology of 60% or more, 65% or more, 70% or more, 75% or more, 80% or more, 85% or more, 90% or more, 91% or more, 92% or more, 93% or more, 94% or more, 95% or more, 96% or more, 97% or more, 98% or more, 99% or more, 99.5% or more, or 99.9% or more (for example, 60% to 99.5%, 70% to 99.5%, 80% to 99.5%, 85% to 99.5%, 90% to 99.5%, 91% to 99.5%, 92% to 99.5%, 93% to 99.5%, 94% to 99.5%, 95% to 99.5%, 96% to 99.5%, 97% to 99.5%, 98% to 99.5%, or 99% to 99.5%) with the specific nucleic acid sequence or amino acid sequence or essentially comprising it, and maintaining the original function and/or desired function. In the present description, the original function may be an L-histidine export function (in case of the amino acid sequence), or a function of encoding a protein having an L-histidine export function (in case of the nucleic acid sequence), and the desired function may mean a function of increasing or giving L-histidine producing ability of a microorganism.

In the nucleic acid sequence described in the present description, various modifications may be made to a coding region within a range that does not change the amino acid sequence and/or function of a protein expressed from the coding region, in consideration of a preferred codon in a microorganism to express the protein (L-histidine export protein) by degeneracy of the codon.

In the present application, the term, `homology' or 'identity' means a degree to which two given amino acid sequences or base sequences are related and may be expressed as a percentage. The terms, homology and identity may be often used interchangeably.

The sequence homology or identity of the conserved polynucleotide or polypeptide is determined by a standard array algorithm, and a default gap penalty established by the used program may be used together. Substantially, the homologous or identical sequence may be generally hybridized under moderate or high stringent conditions along at least about 50%, 60%, 70%, 80% or 90% of the entire sequence or full-length. It is obvious that hybridization also includes a polynucleotide containing a common codon or a codon considering codon degeneracy in a polynucleotide.

Whether any two polynucleotide or polypeptide sequences have homology, similarity, or identity, may be determined using for example, a known computer algorithm such as "FASTA" program using Pearson et al (1988) [Proc. Natl. Acad. Sci. USA 85]: 2444. Otherwise, it may be determined using Needleman-Wunsch algorithm (Needleman and Wunsch, 1970, J. Mol. Biol. 48: 443-453), performed in Needleman program of EMBOSS package (EMBOSS: The European Molecular Biology Open Software Suite, Rice et al., 2000, Trends Genet. 16: 276-277) (version 5.0.0 or next version) (including GCG 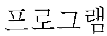 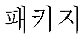 (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, the homology, similarity, or identity may be determined using BLAST, or ClustalW of National Biotechnology Information Database Center.

The homology, similarity, or identity of the polynucleotide or polypeptide may be determined by comparing sequence information using GAP computer program such as for example, Needleman et al. (1970), J Mol Biol. 48:443, as known in for example, Smith and Waterman, Adv. Appl. Math (1981) 2:482. In summary, the GAP program may be defined as a value of dividing the total number of symbols in the shorter of two sequences by the number of similarly arranged symbols (i.e., nucleotide or amino acid). The default parameter for the GAP program may include (1) a binary comparison matrix (containing values of 1 for identity and 0 for non-identity) and a weighted comparison matrix of Gribskov et al(1986) Nucl. Acids Res. 14: 6745 as disclosed by Schwartz and Dayhoff, eds., Atlas Of Protein Sequence And Structure, National Biomedical Research Foundation, pp. 353-358 (1979) (or EDNAFULL (EMBOSS version of NCBI NUC4.4) substitution matrix); (2) 3.0 penalty for each gap and additional 0.10 penalty for each symbol in each gap (or gap opening penalty 10, gap extension penalty 0.5); and (3) no penalty for end gaps.

In addition, whether any two polynucleotide or polypeptide sequences have homology, similarity, or identity may be confirmed by comparing sequences by a southern hybridization experiment under the defined stringent condition, and the defined appropriate hybridization condition is within the corresponding technology range, and it may be determined by a method known to those skilled in the art (for example, J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

Introduction or transformation of a gene encoding the L-histidine export protein may be performed by appropriately selecting a known transformation method using a common expression vector by those skilled in the art. In the present description, the term "transformation" means introducing an expression vector comprising a polynucleotide encoding a target protein (L-histidine export protein) into a host microorganism so that the protein encoded by the polynucleotide can be expressed in the host cell. As long as the transformed polynucleotide can be expressed in the host microorganism, it may be located as inserted in chromosome of the host microorganism and/or located outide the chromosome. As long as the polynucleotide can be introduced and expressed into a host microorganism, the introduced form is not limited. For example, the polynucleotide may be introduced into a host microorganism in a form of an expression cassette which is a gene structure comprising all elements required for being autonomously expressed. The expression cassette may commonly comprise expression regulatory elements such as a promoter, a transcription termination signal, a ribosome binding site and/or a translation termination signal and the like which are operably linked to the polynucleotide. The expression cassette may be in a form of an expression vector capable of self-replicating. In addition, the polynucleotide may be introduced into a host cell in its own form and operably linked to a sequence required for expression in a host cell. In the above, the term "operably linked" may mean that the expression regulatory elements (e.g., promoter) and polynucleotide are functionally linked so that the expression regulatory elements perform transcription regulation (e.g., transcription initiation) of the polynucleotide encoding a target protein (L-histidine export protein). Operable linking may be performed using a known gene recombination technology in the art, and for example, it may be performed by common site-specific DNA cleavage and ligation, but not limited thereto.

The method for transforming the polynucleotide into a host microorganism can be performed by any method for introducing a nucleic acid into a cell (microorganism), and a transformation technology known in the art may be appropriately selected and performed according to the host microorganism. As the known transformation method, electroporation, calcium phosphate (CaPO₄) precipitation, calcium chloride (CaCl₂) precipitation, microinjection, polyethylene glycol (PEG) precipitation (polyethylene glycol-mediated uptake), DEAE-dextran method, cationic liposome method, lipofection, lithium acetate-DMSO method, and the like may be exemplified, but not limited thereto.

Insertion of the gene into the host cell genome (chromosome) may be performed by appropriately selecting a known method by those skilled in the art, and for example, it may be performed using for example, RNA-guided endonuclease system (for example, at least one selected from the group consisting of (a) RNA-guided endonuclease (e.g., Cas9 protein, etc.), its encoding gene, or a vector comprising the gene; and (b) a mixture (for example, a mixture of RNA-guided endonuclease protein and guide RNA, etc.), a complex (for example, ribonucleic acid fusion protein (RNP), a recombinant vector (for example, a vector comprising RNA-guided endonuclease encoding gene and guide RNA encoding DNA together, etc.) and the like which comprises guide RNA (e.g., single guide RNA (sgRNA), etc.), its encoding DNA, or a vector comprising the DNA, but not limited thereto.

Another embodiment provides a nucleic acid molecule encoding the L-histidine export protein. In one embodiment, the nucleic acid molecule may be a nucleic acid molecule comprising the nucleic acid sequence of SEQ ID NO: 64 and/or SEQ ID NO: 65, or SEQ ID NO: 14; SEQ ID NO: 66 and/or SEQ ID NO: 67, or SEQ ID NO: 43; or SEQ ID NO: 68 and/or SEQ ID NO: 69, or SEQ ID NO: 46, or consisting of the sequence.

Other embodiment provides a recombinant vector (expression vector) comprising the nucleic acid molecule.

Other embodiment provides a recombinant cell comprising the nucleic acid molecule or recombinant vector.

One embodiment provides a composition for producing L-histidine, a composition for increasing L-histidine production, or a composition for preparing an L-histidine producing microorganism, comprising a nucleic acid molecule encoding an L-histidine export protein, a recombinant vector comprising the nucleic acid molecule, or a cell comprising the nucleic acid molecule or the recombinant vector.

Other embodiment provides a method for preparing an L-histidine producing microorganism, or a method for enhancing and/or giving L-histidine producing ability of the microorganism, comprising modifying a microorganism to express an L-histidine export protein. The modifying a microorganism to express an L-histidine export protein may be performed by introducing a gene encoding an L-histidine export protein into the microorganism, or transforming the microorganism with a gene encoding an L-histidine export protein.

The L-histidine export protein, gene encoding it and microorganism are as described above.

In the present description, the term "vector" means a DNA preparation containing a bas sequence of a polynucleotide encoding the target protein operably linked to an appropriate regulatory sequence to enable expression of a target protein in a suitable host. The regulatory resequence may comprise a promoter which can initiate transcription, any operator sequence for regulating transcription, a sequence encoding an appropriate mRNA ribosome binding site, and/or a sequence regulating termination of transcription and/or translation. The vector may be expressed independently of the genome of the host microorganism or integrated into the genome of the host microorganism, after being transformed into a suitable host microorganism.

The vector usable in the present description is not particularly limited as long as it can be replicated in a host cecll, and may be selected from all vectors commonly used. The example of the commonly used vector may include natural or recombinant plasmids, cosmids, viruses, bacteriophages and the like. For example, as the vector, pWE15, M13, MBL3, MBL4, IXII, ASHII, APII, t10, t11, Charon4A, and Charon21A and the like may be used as the phage vector or cosmid vector, and pBR-based, pUC-based, pBluescriptII-based, pGEM-based, pTZ-based, pCL-based and pET-based and the like may be used as the plasmid vector. Specifically, pDZ, pACYC177, pACYC184, pCL, pECCG117, pUC19, pBR322, pMW118, pCC1BAC vectors, and the like may be exemplified, but not limited thereto.

The vector usable in the present description may be a known expression vector and/or a vector for insertion in host cell chromosome of a polynucleotide. Insertion of the polynucleotide into host cell chromosome may be conducted by any method known in the art, for example, homologous recombination, but not limited thereto. The vector may further comprise a selection marker for confirming the insertion in the chromosome. The selection marker is for selecting a cell transformed with the vector, that is, confirming insertion of the polynucleotide, and it may be selected and used among genes conferring selectable phenotypes such as drug resistance, auxotrophy, resistance to cytotoxic agents or expression of surface proteins. In an environment treated with a selective agent, only cells expressing the selectable marker survive or exhibit other expression traits, and thus transformed cells can be selected.

Other embodiment provides a method for production of L-histidine, comprising culturing the L-histidine producing microorganism in a medium. The method may further comprise recovering L-histidine from the cultured microorganism, medium or both of them, after the culturing.

In the method, the culturing the microorganism is not particularly limited, but may be performed by known batch culture method, continuous culture method, fed-batch culture method, and the like. Then, the culturing condition, is not particularly limited, but may adjust titration pH (for example, pH 5 to 9, specifically, pH 6 to 8, most specifically, pH 6.8) using a basic compound (e.g.: sodium hydroxide, potassium hydroxide, or ammonia) or an acidic compound (e.g.: phosphate or sulfate), and the aerobic condition can be maintained by introducing oxygen or an oxygen-containing gas mixture into the culture. The culturing temperature may be maintained at 20 to 45°C, or 25 to 40°C, and it may be cultured for about 10 to about 160 hours, about 10 hours to 96 hours, about 10 hours to 48 hours, or about 10 hours to 36 hours, but not limited thereto. The L-histidine produced by the culturing may be secreted in the medium or remain in the cells.

The medium usable for the culturing may use at least one selected from the group consisting of sugars and carbohydrates (e.g.: glucose, sucrose, lactose, fructose, maltose, molasse, starch and cellulose), oils and fats (e.g.: soybean oil, sunflower oil, peanut oil, and coconut oil), fatty acids (e.g.: palmitic acid, stearic acid and linoleic acid), alcohols (e.g.: glycerol and ethanol), organic acids (e.g.: acetic acid) and the like individually, or mix and use at least two kinds thereof as a carbon source, but not limited thereto. As a nitrogen source, at least one selected from the group consisting of nitrogen-containing organic compounds (e.g.: peptone, yeast extract, meat juice, malt extract, corn steep liquor, soybean meal powder and urea), inorganic compounds (e.g.: ammonium sulfate, ammonium chloride, ammonium phosphate, ammonium carbonate and ammonium nitrate) and the like may be used individually or at least 2 kinds thereof may be mixed and used, but not limited thereto. As a phosphorus source, at least one selected from the group consisting of potassium dihydrogen phosphate, dipotassium hydrogen phosphate, salts containing sodium corresponding thereto, and the like may be used individually or at least 2 kinds thereof may be mixed and used, but not limited thereto. In addition, the medium may comprise an essential growth-promoting substance such as other metal salts (e.g.: magnesium sulfate or iron sulfate), amino acids and/or vitamins and the like.

The recovering L-histidine may be collecting a desired amino acid from a medium, culturing solution or microorganism using an appropriate method known in the art according to the culture method. For example, the recovering may be performed by at least one method selected from centrifugation, filtration, anion exchange chromatography, crystallization, HPLC and the like. The method for recovering L-histidine may further comprise purification, before, at the same time as, or after the recovering.

### [ADVANTAGEOUS EFFECTS]

By expressing the L-histidine export gene provided in the present description in a microorganism having L-histidine producing ability, compared to the parent strain in which the gene is not expressed, the L-histidine production can be dramatically improved, so not only L-histidine can be more effectively produced, but also it can also contribute to industrial large-scale production of L-histidine.

### [MODE FOR INVENTION]

Hereinafter, the present application will be described in more detail by examples. However, these examples are intended to illustratively describe the present application, but the scope of the present application is not limited by these examples.

### Example 1. Foreign histidine export gene search and candidate selection

L-histidine is mainly calssified as a basic amino acid among amino acid categories, but it is also classified as an aromatic amino acid or a branched chain amino acid. In order to select a protein candidate having L-histidine-specific export ability, as a result of PSI-BLAST search using an amino acid sequence of an export protein (LysE (Arch Microbiol 180: 155-160), Wex (Korean Patent No. 10-1968317), BrnFE (Arch Microbiol 180: 155-160)) for an amino acid of each category (basic amino acid: L-lysine, aromatic amino acid: Trp, branched chain amino acid: isoleucine) as a query sequence, based on NCBI and Kegg database, candidate genes predicted to be membrane proteins likely to release L-histidine and microorganisms possessing them were selected.

Among them, considering the biosafety level applicable to the producing strain and the securing possibility, as shown in Table 1 below, proteins of one kind based on LysE, 3 kinds based on Wex and 2 kinds based on BrnFE, genes encoding them, and microorganisms comprising them were selected:

**[Table 1]**

| | L-histidine exporter candidate list | | | | | | |
|---|---|---|---|---|---|---|---|
| | No. | Strain | Protein Ref Seq. | gDNA Ref Seq. | Biosafety | Amino acid sequence | Nucleic acid sequence |
| Wex-based | 1 | *Herbaspirillum aquaticum* (KCTC42001) | WP_088757482.1 | NZ_NJGV0100 0035.1 | 1 | SEQ ID NO: 1 | SEQ ID NO: 2 |
| | 2 | *Cupriavidus pinatubonensis* (KCTC22125) | WP_041680244.1 | CP000091.1 | 1 | SEQ ID NO: 3 | SEQ ID NO: 4 |
| | 3 | *Kluyvera cryocrescens* (KCTC2580) | WP_052283291.1 | NZ_LGHZ0100 0014.1 | 1 | SEQ ID NO: 5 | SEQ ID NO: 6 |
| LysE-based | 4 | *Corynebacterium stationis* (ATCC6872) | WP_066837457.1 | CP014279.1 | 1 | SEQ ID NO: 7 | SEQ ID NO: 8 |
| BrnFE-based | 5 | *Leucobacter salsicius* (KCTC19904) | WP_026139602.1 | NZ_AOCN0100 0022.1 | 1 | SEQ ID NO: 9 | SEQ ID NO: 11 |
| | | | WP_083879221.1 | | | SEQ ID NO: 10 | |
| | 6 | *Dermabacter vaginalis* (KCTC39585) | WP_065248528.1 (DvaF) | NZ_CP012117. 1 | 1 | SEQ ID NO: 12 | SEQ ID NO: 64 |
| | | | WP_065248527.1( DvaE) | | | SEQ ID NO: 13 | SEQ ID NO: 65 |
| | | | DvaFE | | | SEQ ID | SEQ ID |
| | | | | | | NO: 12 and 13 | NO: 14 (DvaFE operon) |

(In Table 1 above, the biosafety is based on the microbial pathogenicity index (level 1~4) defined by Centers for Disease Control and Prevention in U.S. (the lower the level, the safer)

### Example 2. Construction of vector introduced with foreign L-histidine export gene candidate and recombinant strain of the genus Corynebacterium introduced with this

Six kinds of vectors for introducing 6 kinds of the foreign L-histidine export gene candidates selected in Example 1 into a strain of the genus *Corynebacterium* were constructed.

In order to introduce the foreign L-histidine export gene candidates, NCgl2131 gene among genes encoding transposon of *Corynebacterium glutamicum* was used as an insertion site (Journal of Biotechnology 104, 5-25 Jorn Kalinowski et al, 2003). In addition, the foreign L-histidine export gene candidates were designed to be expressed under a promoter of the Corynebacterium-derived gapA gene (hereinafter, PgapA, SEQ ID NO: 15).

In order to substitute the NCgl2131 gene with the exporter genes, an NCg12131-deleted and target gene-inserted vector was constructed. In order to construct the vector, using chromosome of *Corynebacterium glutamicum* strain ATCC13032 as a template, using a primer pair of SEQ ID NO: 16 and SEQ ID NO: 17, and SEQ I D NO: 18 and SEQ ID NO: 19, respectively, PCR was performed, respectively. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, DNA fragments of del-N2131L (SEQ ID NO: 20) of 531bp and del-N2131R (SEQ ID NO: 21) of 555bp were obtained. The obtained DNA products were purified using PCR Purification kit of QIAGEN company, and then cloning was performed using pDZ vector (Korean Patent No. 10-0924065) and TaKaRa's Infusion Cloning Kit, and thereby, the vector for NCgl2131 gene deletion and target gene insertion was constructed.

The base sequence information of the gene encoding *Herbaspirillum aquaticum-*derived protein (hereinafter, Haq, SEQ ID NO: 1) (hereinafter, *haq,* SEQ ID NO: 2) was obtained from National Institutes of Health GenBank (NIH GenBank). In order to amplify *haq,* using chromosome DNA of the *Herbaspirillum aquaticum* strain (KCTC42001) as a template, using the primer pair of SEQ ID NO: 22 and SEQ ID NO: 23, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *haq* fragments of 977bp comprising *haq* (SEQ ID NO: 2) of 945bp were obtained. In order to obtain PgapA fragments connectable to *haq,* using chromosome of ATCC13032 as a template, using the primer pair of SEQ ID NO: 24 and SEQ ID NO: 25, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained.

The base sequence information of the gene encoding *Cupriavidus pinatubonensis*-derived protein (hereinafter, Cpi, SEQ ID NO: 3) (hereinafter, *cpi,* SEQ ID NO: 4) was obtained from National Institutes of Health GenBank (NIH GenBank). In order to amplify *cpi* derived from *Cupriavidus pinatubonensis*, using chromosome DNA of the *Cupriavidus pinatubonensis* strain (KCTC22125) as a template, using the primer pair of SEQ ID NO: 24 and SEQ ID NO: 25, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *cpi* fragments of 977bp comprising *cpi* (SEQ ID NO: 4) of 945bp were obtained. In order to obtain PgapA fragments connectable to *cpi,* using chromosome of ATCC13032 as a template, using the primer pair of SEQ ID NO: 22 and SEQ ID NO: 26, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *cpi* fragments and PgapA fragments, and pDZΔN2131 vector cleaved with ScaI restriction enzyme were cloned using Gibson assembly method, to obtain a recombinant plasmid, and this was named pDZΔN2131-PgapA-Cpi.

The base sequence information of the gene encoding *Kluyvera cryocrescens-*derived protein (hereinafter, Kcr, SEQ ID NO: 5) (hereinafter, *kcr,* SEQ ID NO: 6) was obtained from National Institutes of Health GenBank (NIH GenBank). In order to amplify *kcr* derived from *Kluyvera cryocrescens*, using chromosome DNA of the *Kluyvera cryocrescens* strain (KCTC2580) as a template, using the primer pair of SEQ ID NO: 29 and SEQ ID NO: 30, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *kcr* fragments of 914bp comprising *kcr* (SEQ ID NO: 6) of 882bp were obtained. In order to obtain PgapA fragments connectable to *kcr,* using chromosome of ATCC13032 as a template, using the primer pair of SEQ ID NO: 24 and SEQ ID NO: 31, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *kcr* fragments and PgapA fragments, and pDZΔN2131 vector cleaved with ScaI restriction enzyme were cloned using Gibson assembly method, to obtain a recombinant plasmid, and this was named pDZΔN2131-PgapA-Kcr.

The base sequence information of the gene encoding *Corynebacterium stationis-*derived protein (hereinafter, Cst, SEQ ID NO: 7) (hereinafter, *cst,* SEQ ID NO: 8) was obtained from National Institutes of Health GenBank (NIH GenBank). In order to amplify *cst* derived from *Corynebacterium stationis*, using chromosome DNA of the *Corynebacterium stationis* strain (KCTC6872) as a template, using the primer pair of SEQ ID NO: 32 and SEQ ID NO: 33, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *cst* fragments of 749bp comprising *cst* (SEQ ID NO: 8) of 717bp were obtained. In order to obtain PgapA fragments connectable to *cst,* using chromosome of ATCC13032 as a template, using the primer pair of SEQ ID NO: 24 and SEQ ID NO: 34, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *cst* fragments and PgapA fragments, and pDZΔN2131 vector cleaved with ScaI restriction enzyme were cloned using Gibson assembly method, to obtain a recombinant plasmid, and this was named pDZΔN2131-PgapA-Cst.

The base sequence information of the operon encoding *Leucobacter salsicius-*derived protein (hereinafter, LsaFE, SEQ ID NO: 9, 10) (hereinafter, *Isa,* SEQ ID NO: 11) was obtained from National Institutes of Health GenBank (NIH GenBank). In order to amplify *Isa* derived from *Leucobacter salsicius*, using chromosome DNA of the *Leucobacter salsicius* strain (KCTC19904) as a template, using the primer pair of SEQ ID NO: 35 and SEQ ID NO: 36, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *Isa* fragments of 1080bp comprising *Isa* (SEQ ID NO: 11) of 1048bp were obtained. In order to obtain PgapA fragments connectable to *Isa,* using chromosome of ATCC13032 as a template, using the primer pair of SEQ ID NO: 24 and SEQ ID NO: 37, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *Isa* fragments and PgapA fragments, and pDZΔN2131 vector cleaved with ScaI restriction enzyme were cloned using Gibson assembly method, to obtain a recombinant plasmid, and this was named pDZΔN2131-PgapA-Lsa.

The base sequence information of the operon encoding *Dermabacter vaginalis-*derived protein (hereinafter, DvaFE, SEQ ID NO: 12, 13) (hereinafter, *dva,* SEQ ID NO: 14) was obtained from National Institutes of Health GenBank (NIH GenBank). In order to amplify *dva* derived from *Dermabacter vaginalis,* using chromosome DNA of the *Dermabacter vaginalis* strain (KCTC39585) as a template, using the primer pair of SEQ ID NO: 38 and SEQ ID NO: 39, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *dva* fragments of 1113bp comprising *dva* (SEQ ID NO: 14) of 1081bp were obtained. In order to obtain PgapA fragments connectable to *dva,* using chromosome of ATCC13032 as a template, using the primer pair of SEQ ID NO: 24 and SEQ ID NO: 40, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *dva* fragments and PgapA fragments, and pDZΔN2131 vector cleaved with ScaI restriction enzyme were cloned using Gibson assembly method, to obtain a recombinant plasmid, and this was named pDZΔN2131-PgapA-Dva.

In order to confirm the L-histidine export ability of the foreign L-histidine export gene candidates, the constructed NCgl2131-deleted vector (pDZΔN2131), and 6 kinds of the foreign L-histidine export gene candidate-introduced vectors (pDZΔN2131-PgapA-Haq, pDZΔN2131-PgapA-Cpi, pDZΔN2131-PgapA-Kcr, pDZΔN2131-PgapA-Cst, pDZΔN2131-PgapA-Lsa, pDZΔN2131-PgapA-Dva) were introduced into *Corynebacterium glutamicum* ATCC13032 strain, respectively. More specifically, the vectors were transformed into the ATCC13032 strain by electroporation, respectively, and through a secondary crossing process, 7 kinds of recombinant strains in which the NCgl2131 gene on chromosome was deleted or substituted with the L-histidine export gene candidate were constructed, and they were named ATCC13032ΔN2131 (N2131 gene was deleted), ATCC13032ΔN2131::Haq (N2131 gene was substituted with *haq*), ATCC13032ΔN2131::Cpi (N2131 gene was substituted with *cpi*), ATCC13032ΔN2131::Kcr (N2131 gene was substituted with *kcr*), ATCC13032ΔN2131::Cst (N2131 gene was substituted with *cst*), ATCC13032ΔN2131::Lsa (N2131 gene was substituted with *Isa*), and ATCC13032ΔN2131::Dva (N2131 gene was substituted with *dva*), respectively.

### Example 3. MIC measurement of strains of the genus Corynebacterium introduced with foreign L-histidine export gene candidate

In order to confirm possession of the L-histidine export ability activity of 7 kinds of the recombinant *Corynebacterium glutamicum* strains (ATCC13032ΔN2131, ATCC13032ΔN2131::Haq, ATCC13032ΔN2131::Cpi, ATCC13032ΔN2131::Kcr, ATCC13032ΔN2131::Cst, ATCC13032ΔN2131::Lsa, and ATCC13032ΔN2131::Dva) constructed in Example 2, a minimum inhibitory concentration (MIC) experiment using L-histidine was performed. After culturing 7 kinds of the strains in a minimal liquid medium at 30°C for 24 hours, they were diluted to 1 × 10³ and 1 × 10⁴ cells and cultured by spotting in a minimal solid medium added with L-histidine. The used minimal solid medium composition was as follows:

### Minimal medium (pH 7.2)

Glucose 10g, KH₂PO₄ 1 g, K₂HPO₄ 2 g, MgSO₄ 7H₂O 0.4 g, urea 2 g, (NH₄)₂SO₄ 5 g, NaCl 0.5 g, nicotinamide 5 *µg*, calcium-pantothenic acid 0.1 *µg*, biotin 0.2 *µg*, thiamine HCl 3 *µg*, Trace elements solution* 1ml (based on distilled water 1 liter), 20g Agar

### *Trace elements solution

Na₂B₄O₇ 10H₂O 0.09 g, (NH₄)₆Mo₇O₂₇4H₂O 0.04 g, ZnSO₄ 7H₂O 0.01 g, CuSO₄ 5H₂O 0.27 g, MnCl₂ 4H₂O 0.01 g, FeCl₃ 6H₂O 1 g, CaCl₂ 0.01 g (based on distilled water 1 liter)

For the minimal inhibitory concentration experiment, 1 g/L of L-histidine was added to the minimal solid medium, and cell growth was observed after 48 hours, and the result was shown in Table 2 below:

**[Table 2]**

| Degree of growth of strains of the genus *Corynebacterium* introduced with a foreign L-histidine export gene candidate in minimal medium comprising L-histidine | | |
|---|---|---|
| Strain | Minimal medium comprising no L-histidine | Minimal medium comprising L-histidine 1 g/L |
| ATCC13032ΔN2131 | ++++ | + |
| ATCC13032Δ2131::Haq | ++++ | + |
| ATCC13032ΔN2131::Cpi | ++++ | + |
| ATCC13032ΔN2131::Kcr | ++++ | + |
| ATCC13032ΔN2131::Cst | ++++ | + |
| ATCC13032ΔN2131::Lsa | ++++ | + |
| ATCC13032ΔN2131::Dva | ++ | +++ |

(In Table 2, the number of + indicates the relative growth degree of strains, and each represents the following:
+: single colony is not formed, but heavy (a type that does not grow as a single colony but grows in aggregation) is formed;
++: heavy is formed and single colonies less than 5 are formed;
+++: heavy is formed and single colonies less than 50 are formed;
++++: heavy is formed indistinguishable from single colony)

As shown in Table 2, all thes trains except for ATCC13032ΔN2131::Dva strain grew smoothly in a minimal medium comprising no L-histidine. However, in the minimal medium comprising 1 g/L of L-histidine, the growth of the strains in which most of the L-histidine export candidate genes were introduced was insignificant, and only the ATCC13032ΔN2131::Dva strain in which the *Dermabacter vaginalis-derived* gene was introduced showed growth superior to ATCC13032ΔN2131. This shows that the introduced *Dermabacter vaginalis-derived* protein may have the L-histidine export ability even in a medium comprising L-histidine above the minimal inhibitory concentration.

From this, *Dermabacter vaginalis-derived* protein Dva was selected as a protein which imparts resistance to L-histidine above the minimal inhibitory concentration and has export ability specific to L-histidine.

### Example 4. Construction of strain introduced with Dermabacter vaginalis-derived gene based on Corynebacterium-derived L-histidine producing strain KCCM 80179 and evaluation of L-histidine producing ability

In order to confirm the L-histidine export ability of *Dermabacter vaginalis-*derived protein Dva, the *Dermabacter vaginalis-derived* gene *dva* was introduced into the L-histidine producing strain KCCM 80179 (Korean Patent Application No. 10-2019-004693414-682).

For this, the vectors pDZΔN2131, and pDZΔN2131-PgapA-Dva constructed in Example 2 were transformed by electroporation, and through a secondary crossing process, 2 kinds of strains in which the NCgl2131 gene on chromosome was deleted or substituted with the L-histidine export gene candidate (*dva*) were constructed, and they were named KCCM 80179ΔN2131 (NCgl2131 gene was deleted) and KCCM 80179ΔN2131-PgapA-Dva (NCgl2131 gene was substituted with *dva*), respectively.

In order to confirm the L-histidine producing ability of the constructed KCCM 80179ΔN2131 and KCCM 80179ΔN2131-PgapA-Dva strains, they were cultured by the following method: KCCM 80179ΔN2131 and KCCM 80179ΔN2131-PgapA-Dva strains were cultured in an activation medium for 16 hours, and then each strain was inoculated into a 250 mℓ corner-baffle flask containing 25 mℓ of a seed medium, and cultured with shaking at 200 rpm. Then, 1 mℓ of the seed culturing solution was inoculated into a 250 mℓ corner-baffle flask containing 25 mℓ of a production medium and cultured with shaking at 200 rpm, at 30 °C for 48 hours. The medium composition used for culturing was as follows:

### <Activation medium>

Meat juice 1%(w/v), polypeptone 1%(w/v), sodium chloride 0.5%(w/v), yeast extract 1%(w/v), agar 2%(w/v), pH 7.2

### <Seed medium>

Glucose 5%(w/v), bactopeptone 1%(w/v), sodium chloride 0.25%(w/v), yeast extract 1%(w/v), urea 0.4%(w/v), pH 7.2

### <Production medium>

Glucose 10%(w/v), ammonium sulfate 2%(w/v), potassium phosphatemonobasic 0.1%(w/v), magnesium sulfate heptahydrage 0.05%(w/v), CSL (corn steep liquor) 2.0%(w/v), biotin 200 *µg*/L, calcium carbonate, pH 7.2,

After completing the culturing, the L-histidine production (histidine content in the medium) was measured by HPLC, and the result was shown in the following Table 3:

**[Table 3]**

| L-histidine production of strains introduced with KCCM 80179-derived *Dermabacter vaginalis-derived* gene | | | |
|---|---|---|---|
| | **Cell OD**₆₀₀ | **Used glucose (g/L)** | **Histidine production (g/L)** |
| KCCM 80179 | 51.4 | 100 | 14.1 |
| KCCM 80179ΔN2131 | 51.6 | 100 | 13.9 |
| KCCM 80179ΔN2131-PgapA-Dva | 42.6 | 100 | 17.1 |

As shown in Table 3, it was confirmed that the NCg12131-delted strain had the L-histidine producing ability at the level equivalent to a parent strain, KCCM 80179 strain, whereas the KCCM 80179ΔN2131-PgapA-Dva strain in which the *Dermabacter vaginalis*-derived gene was introduced had the L-histidine producing ability increased by 23% and 21%, respectively, compared to the NCgl2131-deleted strain and the parent strain, KCCM 80179 strain. Through the result of Examples 3 and 4, it was confirmed that not only resistance to the L-histidine concentration above the minimal inhibitory concentration was increased, but also the L-histidine producing ability was largely increased, through introduction of the *Dermabacter vaginalis*-derived gene. This result demonstrates that the *Dermabacter vaginalis*-derived protein is an L-histidine export protein capable of exporting L-histidine specifically.

### Example 5. Additional securing of Dermabacter vaginalis-derived L-histidine exporter-like protein

As the L-histidine export ability of the *Dermabacter vaginalis-derived* protein was confirmed in Examples 3 and 4, in order to additionally secure a similar protein having high amino acid sequence homology with the protein, using the sequence of DvaF (SEQ ID NO: 12) among DvaFE as a query, BLAST search was performed (See Table 4).

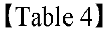

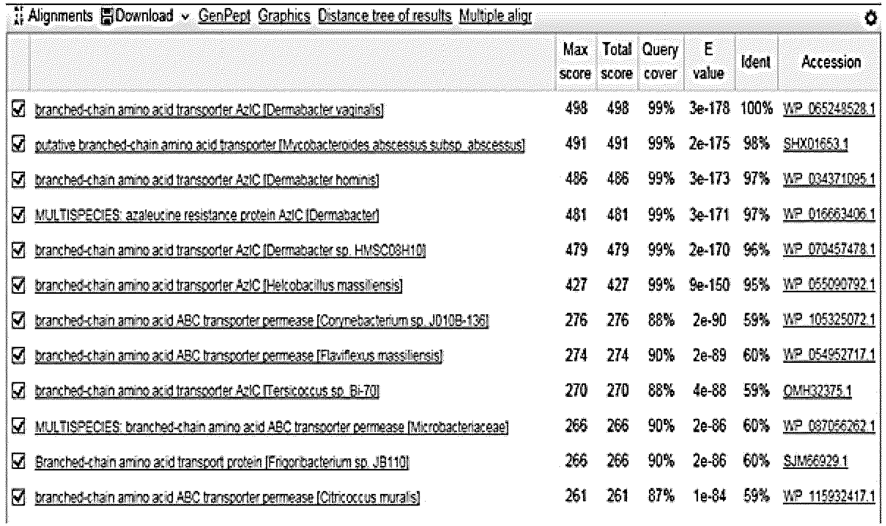

As as result of the BLAST search, 2 kinds of L-histidine exporters which showed at least 60% sequence homology and did not belong to the genus *Dermabacter* were additionally selected, and were shown in Table 5 as follows:

**[Table 5]**

| | L-histidine exporter additional candidate list | | | | | | |
|---|---|---|---|---|---|---|---|
| | No. | Strain | Protein Ref Seq. | gDNA Ref Seq. | Biosafet y | Protein sequence | Gene sequence |
| DvaFE-based | 7 | *Helcobacillus massiliensis* | WP_055090792.1 (HmaF) | NZ_CYU G0100001 7.1 | 2 | SEQ ID NO: 41 (having 95% homology with SEQ ID NO: 12) | SEQ ID NO: 66 |
| | | | WP_055090293.1 (HmaE) | | | SEQ ID NO: 42 (having 95% homology with SEQ ID NO: 13) | SEQ ID NO: 67 |
| | | | HmaFE | | | SEQ ID NO: 41 and 42 | SEQ ID NO: 43 (HmaFE operon) |
| | 8 | *Mycobacterium abscessus subsp. abscessus* | SHX01622.1 (MabF) | FSEE0100 0013.1 | 1 | SEQ ID NO: 44 (having 98% homology with SEQ ID NO: 12) | SEQ ID NO: 68 |
| | | | SHX01653.1 | | | SEQ ID NO: | SEQ ID NO: |
| | | | (MabE) | | | 45(having 99% homology with SEQ ID NO: 13) | 69 |
| | | | MabFE | | | SEQ ID NO: 44 and 45 | SEQ ID NO: 46 (MabFE operon) |

### Example 6. Construction of vectors introduced with additional foreign L-histidine export gene candidates

Two kinds of vectors for introducing 2 kinds of the L-histidine export gene candidates additionally selected in Example 5 into the strain of the genus *Corynebacterium* were constructed. As same as Example 2, the NCgl2131 gene was used as a deletion site, and PgapA was used as a promoter.

The base sequence information of the operon encoding *Helcobacillus massiliensis*-derived protein (hereinafter, HmaFE, SEQ ID NO: 41, 42) (hereinafter, *hma,* SEQ ID NO: 43) was obtained from National Institutes of Health GenBank (NIH GenBank). In order to obtain *haq* DNA, DNA was synthesized using gene synthesis service of Bionics company ( 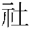 ). In order to amplify the synthesized DNA, using the primer pair of SEQ ID NO: 47 and SEQ ID NO: 48, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *hma* fragments of 1113bp comprising *hma* (SEQ ID NO: 43) of 1081bp were obtained. In order to obtain PgapA fragments connectable to *hma,* using chromosome of ATCC13032 as a template, using the primer pair of SEQ ID NO: 24 and SEQ ID NO: 49, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *hma* fragments and PgapA fragments, and pDZΔN2131 vector cleaved with ScaI restriction enzyme were cloned using Gibson assembly method, to obtain a recombinant plasmid, and this was named pDZΔN2131-PgapA-Hma.

The base sequence information of the operon encoding *Mycobacterium abscessus subsp. abscessus*-derived protein (hereinafter, MabFE, SEQ ID NO: 44, 45) (hereinafter, *mab,* SEQ ID NO: 46) was obtained from National Institutes of Health GenBank (NIH GenBank). In order to obtain *mab* DNA, DNA was synthesized using gene synthesis service of Bionics company ( 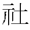 ). In order to amplify the synthesized DNA, using the primer pair of SEQ ID NO: 50 and SEQ ID NO: 51, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *mab* fragments of 1113bp comprising *mab* (SEQ ID NO: 46) of 1081bp were obtained. In order to obtain PgapA fragments connectable to *mab,* using chromosome of ATCC13032 as a template, using the primer pair of SEQ ID NO: 24 and SEQ ID NO: 52, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PgapA fragments of 441bp comprising PgapA (SEQ ID NO: 15) of 409bp were obtained. The obtained *mab* fragments and PgapA fragments, and pDZΔN2131 vector cleaved with ScaI restriction enzyme were cloned using Gibson assembly method, to obtain a recombinant plasmid, and this was named pDZΔN2131-PgapA-Mab.

### Example 7. Construction of strain introduced with Helcobacillus massiliensis-derived, Mycobacterium abscessus subsp. Abscessus-derived genes based on L-histidine producing strain KCCM 80179 and evaluation of L-histidine producing ability

In order to confirm whether the *Helcobacillus massiliensis*-derived protein Hma and *Mycobacterium abscessus subsp. abscessus*-derived protein Mab have the L-histidine export ability, *hma* and *mab* were introduced into the L-histidine producing strain KCCM 80179 strain, respectively.

For this, the vectors pDZΔN2131-PgapA-Hma and pDZΔN2131-PgapA-Mab constructed in Example 6 were transformed by electroporation, and through a secondary crossing process, 2 kinds of strains in which the NCgl2131 gene on chromosome was substituted with the L-histidine export gene candidate were constructed, and they were named KCCM 80179ΔN2131-PgapA-Hma (NCgl2131 gene was substituted with *hma*) and KCCM 80179ΔN2131-PgapA-Mab (NCgl2131 gene was substituted with *mab*), respectively.

In order to confirm the L-histidine producing ability of the constructed KCCM 80179ΔN2131-PgapA-Hma and KCCM 80179ΔN2131-PgapA-Mab strains, the strains were cultured by the method performed in Example 4 and the L-histidine production was measured. For the KCCM 80179ΔN2131 strain and KCCM 80179ΔN2131-PgapA-Dva strain constructed in Example 4 as a control group, culturing and measurement of the L-histidine production (histidine content in the medium) were performed by the same method. The obtained result was shown in Table 6.

**[Table 6]**

| L-histidine production of strains introduced with KCCM 80179-derived *Helcobacillus massiliensis-derived* gene or *Mycobacterium abscessus subsp. abscessus-*derived gene | | | |
|---|---|---|---|
| | **OD** | **Used glucose (g/L)** | **Histidine Production (g/L)** |
| KCCM 80179 | 51.1 | 100 | 14.1 |
| KCCM 80179ΔN2131 | 50.5 | 100 | 13.9 |
| KCCM 80179ΔN2131-PgapA-Dva | 41.6 | 100 | 17.1 |
| KCCM 80179ΔN2131-PgapA-Hma | 42.8 | 100 | 16.4 |
| KCCM 80179ΔN2131-PgapA-Mab | 44.1 | 100 | 16.1 |

As shown in Table 6, in the KCCM 80179ΔN2131-PgapA-Hma strain and KCCM 80179ΔN2131-PgapA-Mab strain, the L-histidine production was increased by 18% and 15.8%, respectively, compared to the parent strain, KCCM 80179 strain. Such a result shows that the *Helcobacillus massiliensis-derived* protein and *Mycobacterium abscessus subsp. abscessus*-derived protein could be also selected as an L-histidine exporter.

### Example 8. Construction of strain introduced with Dermabacter vaginalis-derived, Helcobacillus massiliensis-derived, Mycobacterium abscessus subsp. abscessus-derived genes based on L-histidine producing strain CA14-737 and evaluation of L-histidine producing ability

In order to confirm the L-histidine export ability of the *Dermabacter vaginalis-*derived protein Dva, *Helcobacillus massiliensis*-derived protein Hma, and *Mycobacterium abscessus subsp. abscessus*-derived protein Mab once again, they were introduced into the L-histidine producing strain CA14-737 (Korean Patent Application No. 10-2019-004693414-682) derived from wild-type *Corynebacterium glutamicum* in which HisG polypeptide mutation in which feedback restriction by L-histidine was resolved was introduced, and an L-histidine biosynthesis gene was enhanced.

For this, 4 kinds of the vectors constructed in Examples 2 and 6 (pDZΔN2131, pDZΔN2131-PgapA-Dva, pDZΔN2131-PgapA-Hma, pDZΔN2131-PgapA-Mab) were transformed into the CA14-737 strain by electroporation, respectively, and through a secondary crossking process, 4 kinds of strains in which the NCgl2131 gene on chromosome was deleted or substituted with the L-histidine export gene candidate were constructed, and they were named CA14-737ΔN2131, CA14-737ΔN2131-PgapA-Dva, CA14-737ΔN2131-PgapA-Hma, and CA14-737ΔN2131-PgapA-Mab, respectively.

In order to confirm the L-histidine producing ability of the constructed CA14-737ΔN2131, CA14-737ΔN2131-PgapA-Dva, CA14-737ΔN2131-PgapA-Hma, CA14-737ΔN2131-PgapA-Mab strains, they were cultured by the method performed in Example 4, and the L-histidine production (histidine content in the medium) was measured, and the result was shown in Table 7 as follows:

**[Table 7]**

| L-histidine production of strains introduced with CA14-737-derived *Dermabacter vaginalis*-derived gene, *Helcobacillus massiliensis*-derived gene, *Mycobacterium abscessus subsp. abscessus*-derived gene | | | |
|---|---|---|---|
| | **OD** | **Used glucose (g/L)** | **Histidine Production (g/L)** |
| CA14-737 | 89.6 | 100 | 4.0 |
| CA14-737ΔN2131 | 90.1 | 100 | 4.1 |
| CA14-737ΔN2131-PgapA-Dva | 71.8 | 100 | 6.5 |
| CA14-737ΔN2131-PgapA-Hma | 73.8 | 100 | 5.9 |
| CA14-737ΔN2131-PgapA-Mab | 70.1 | 100 | 6.1 |

As shown in Table 7, the CA14-737ΔN2131-PgapA-Dva strain in which the *Dermabacter vaginalis*-derived gene was introduced had the L-histidine production increased by 62.5% compared to a parent strain, CA14-737 strain, and in the CA14-737ΔN2131-PgapA-Hma strain in which the *Helcobacillus massiliensis*-derived gene was introduced, 47.5% was increased, and in the CA14-737ΔN2131-PgapA-Mab strain in which *Mycobacterium abscessus subsp. abscessus*-derived gene was introduced, 52.5% was increased. Through this, it was confirmed once again that all of the *Dermabacter vaginalis*-derived protein, *Helcobacillus massiliensis*-derived protein, and *Mycobacterium abscessus subsp. abscessus*-derived protein were L-histidine exporters capable of exporting L-histidine specifically. Among the constructed recombinant strains, the CA14-737ΔN2131-PgapA-Dva strain (*Corynebacterium glutamicum* CA14-0875) was internationally deposited at Korean Culture Center of Microorganisms (KCCM), an international depository institution under Budapest Treaty, located in Seodaemun-gu, Seoul, Korea on September 21, 2020, and an accession number, KCCM12793P was given.

### Example 9. Construction of vector for expressing Dermabacter vaginalis-derived, Helcobacillus massiliensis-derived, Mycobacterium abscessus subsp. abscessus-derived protein E. coli

Whether the selected L-histidine exporters showed L-histidine producing ability in various strains was confirmed. For this, vectors capable of expressing the *Dermabacter vaginalis-derived, Helcobacillus massiliensis*-derived, *Mycobacterium abscessus subsp. abscessus*-derived proteins, respectively, in E. coli were constructed. Each gene was cloned into pCC1BAC (hereinafter, pBAC, Epicenter corp.), an E. coli expression vector, and it was expressed under yccA promoter of E. coli strain MG1655 (hereinafter, PyccA, SEQ ID NO: 53).

In order to amplify *Dermabacter vaginalis*-derived *dva*, using chromosome DNA of the *Dermabacter vaginalis* strain as a template, using the primer pair of SEQ ID NO: 54 and SEQ ID NO: 55, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *dva* fragments of 1113bp comprising *dva* (SEQ ID NO: 10) of 1081bp were obtained. In order to obtain PyccA fragments connectable to *dva*, using chromosome of MG1655 as a template, using the primer pair of SEQ ID NO: 56 and SEQ ID NO: 57, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PyccA fragments of 132bp comprising PyccA (SEQ ID NO: 53) of 100bp were obtained. The obtained *dva* fragments and PyccA fragments, and pBAC vector cleaved with EcoRI restriction enzyme were cloned using Gibson assembly method, to obtain a recombinant plasmid, and this was named pBAC-PyccA-Dva.

In order to amplify *Helcobacillus massiliensis*-derived *hma*, using pDZΔN2131-PgapA-Hm vector DNA constructed in Example 6 as a template, using the primer pair of SEQ ID NO: 58 and SEQ ID NO: 59, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *hma* fragments of 1113bp comprising *hma* (SEQ ID NO: 43) of 1081bp were obtained. In order to obtain PyccA fragments connectable to *hma,* using chromosome of MG1655 as a template, using the primer pair of SEQ ID NO: 56 and SEQ ID NO: 60, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PyccA fragments of 132bp comprising PyccA (SEQ ID NO: 53) of 100bp were obtained. The obtained *hma* fragments and PyccA fragments, and pBAC vector cleaved with EcoRI restriction enzyme were cloned using Gibson assembly method, to obtain a recombinant plasmid, and this was named pBAC-PyccA-Hma.

In order to amplify *Mycobacterium abscessus subsp. abscessus*-derived *mab*, using pDZΔN2131-PgapA-Mab vector DNA constructed in Example 6 as a template, using the primer pair of SEQ ID NO: 61 and SEQ ID NO: 62, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 2 minutes, 28 times, performing polymerization at 72°C for 5 minutes. As a result, *mab* fragments of 1113bp comprising *mab* (SEQ ID NO: 43) of 1081bp were obtained. In order to obtain PyccA fragments connectable to *mab,* using chromosome of MG1655 as a template, using the primer pair of SEQ ID NO: 56 and SEQ ID NO: 63, PCR was performed. As polymerase for PCR reaction, PfuUltraTM high-reliability DNA polymerase (Stratagene) was used, and PCR conditions were as follows: after repeating denaturation 95°C, 30 seconds; annealing 55°C, 30 seconds; and polymerization 72°C, 1 minutes, 28 times, performing polymerization at 72°C for 5 minutes. As a result, PyccA fragments of 132bp comprising PyccA (SEQ ID NO: 53) of 100bp were obtained. The obtained *mab* fragments and PyccA fragments, and pBAC vector cleaved with EcoRI restriction enzyme were cloned using Gibson assembly method, to obtain a recombinant plasmid, and this was named pBAC-PyccA-Mab.

### Example 10. Construction of strain introduced with Dermabacter vaginalis-derived gene based on E. coli-derived L-histidine producing strain and evaluation of L-histidine producing ability

In order to confirm the L-histidine export ability of 3 kinds of the new histidine exports (Dva, Hma, Mab) based on the E. coli-derived L-histidine producing strain, the 3 kinds of the constructed vectors were introduced into CA14-9003e strain having a previously reported genotype (purR deletion, hisL deletion, hisG^{r;} The directed modification of *Escherichia coli* MG1655 to obtain histidine-producing mutants; Applied Biochemistry and Microbiology, 2013, Vol. 49, No. 2, pp. 130-135) (MG1655⁺ hisG^{r} hisL'_A ΔpurR). For this, the 3 kinds of the vectors constructed in Example 9 (pBAC-PyccA-Dva, pBAC-PyccA-Hma, pBAC-PyccA-Mab) and the pBAC vector were introduced, respectively, thereby constructing CA14-9003e/pBAC, CA14-9003e/pBAC-PyccA-Dva, CA14-9003e/pBAC-PyccA-Hma, and CA14-9003e/pBAC-PyccA-Mab strains.

In order to confirm the L-histidine producing ability of the constructed CA14-9003e/pBAC, CA14-9003e/pBAC-PyccA-Dva, CA14-9003e/pBAC-PyccA-Hma, CA14-9003e/pBAC-PyccA-Mab strains, they were cultured by the following method. The strains were cultured in an LB solid medium (comprising 25*µg*/ml chloramphenicol) for 16 hours, and then each strain was inoculated in a 250 mℓ corner-baffle flask containing 25 mℓ of an LB liquid medium, and they were cultured with shaking at 200 rpm at 37 °C for 20 hours. Then, 1 mℓ of seed culture solution was inoculated in a 250 mℓ corner-baffle flask containing 25 mℓ of the E. coli production medium (Applied Biochemistry and Microbiology, 2013, Vol. 49, No. 2, pp. 130-135), and it was cultured with shaking at 200 rpm, at 37 °C for 48 hours. The medium used for the culturing was as follows:

### <E. coli production medium>

Glucose 4%(w/v), yeast extract 0.2%(w/v), ammonium sulfate 1.6%(w/v), potassium phosphate dibasic trihydrate 0.06%(w/v), iron sulfate heptahydrate 0.0005%(w/v), magnesium sulfate pentahydrate 0.0005%(w/v), calcium carbonate, pH 7.2,
After completing the culturing, the L-histidine production (histidine content in the medium) was measured by HPLC and the result was shown in Table 8 as follows.

**[Table 8]**

| L-histidine production of strains introduced with CA14-9003e-derived *Dermabacter vaginalis*-derived gene, *Helcobacillus massiliensis*-derived gene, *Mycobacterium abscessus subsp. abscessus*-derived gene | | | |
|---|---|---|---|
| | **OD** | **Used glucose (g/L)** | **Histidine production (g/L)** |
| CA14-9003e/pBAC | 23.6 | 40 | 3.2 |
| CA14-9003e/pBAC-PgapA-Dva | 17.6 | 40 | 4.2 |
| CA14-9003e/pBAC-PgapA-Hma | 18.7 | 40 | 3.9 |
| CA14-9003e/pBAC-PgapA-Mab | 18.9 | 40 | 3.8 |

As shown in Table 8, the CA14-9003e/pBAC-PgapA-Dva strain introduced with the *Dermabacter vaginalis*-derived gene had L-histidine production increased by 62.5%, and the CA14-9003e/pBAC-PgapA-Hma strain introduced with the *Helcobacillus massiliensis*-derived gene had L-histidine production increased by 21.9%, and the CA14-9003e/pBAC-PgapA-Mab strain introduced with the *Mycobacterium abscessus subsp. Abscessus*-derived gene had L-histidine production increased by 18.8%. Through this, it was confirmed that all of the *Dermabacter vaginalis*-derived protein, *Helcobacillus massiliensis*-derived protein, and *Mycobacterium abscessus subsp. abscessus*-derived protein operated as an exporter specific to L-histidine in the E. coli L-histidine producing strain.

Through the above result, it was confirmed that it operated as an exporter which exports L-histidine specifically outside cells when a protein with at least 60% homology with the *Dermabacter vaginalis*-derived protein was introduced into a microorganism.

Above, each description and embodiment disclosed in the present description can be also applied to each other description and embodiment. All possible combinations of various elements disclosed in the present description are within the scope of the invention proposed in the present description. In addition, it cannot be said that the scope of the invention in the present description is limited by the specific description described below, and as long as those skilled in the art can recognize or identify many equivalents to the specific embodiment described in the present description, these equivalents are intended to be included in the invention proposed in the present description.

### [Accessin Number]

Name of depository authority: Korean Culture Center of Microorganisms
Accession number: KCCM12793P
Accession date: 20200921

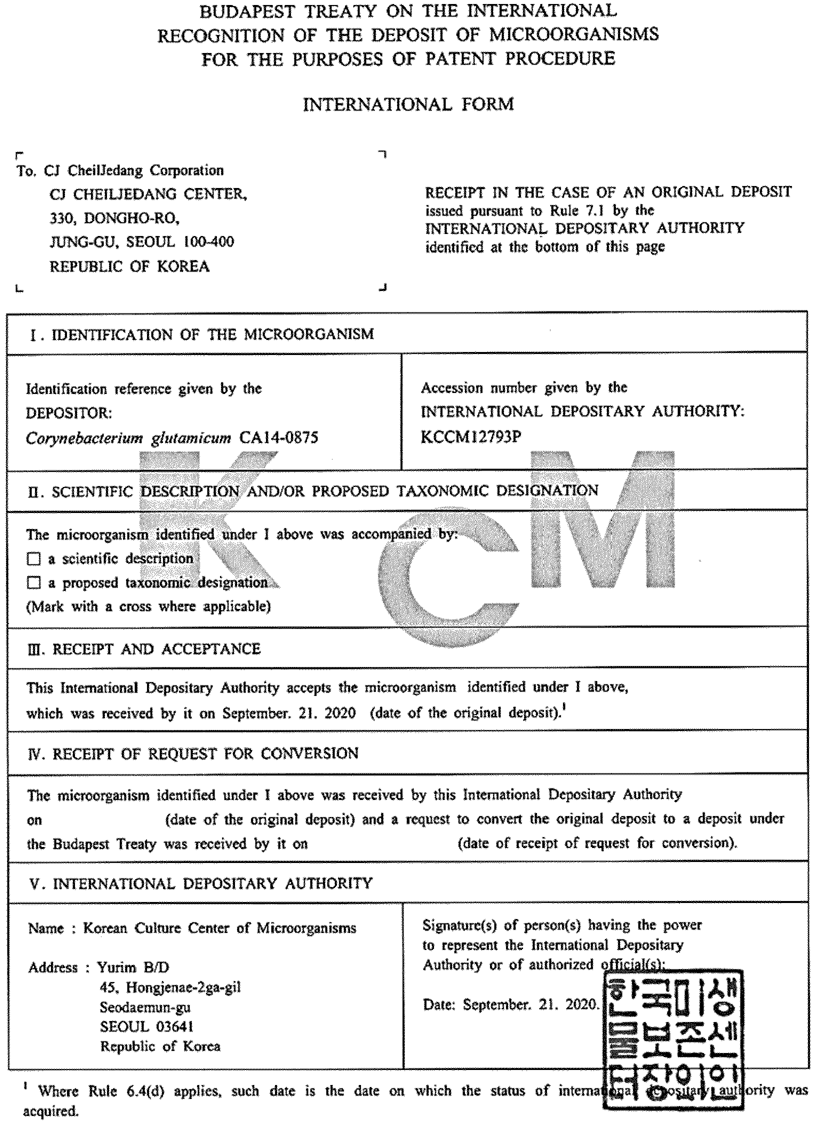
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CheilJedang Corporation
CJ CHEILJEDANG CENTER,
330, DONGHO-RO,
JUNG-GU, SEOUL 100-400
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the
DEPOSITOR:
Corynebacterium glutamicum CA14-0875 | Accession number given by the
INTERNATIONAL DEPOSITARY AUTHORITY:
KCCM12793P
II. SCIENTIFIC DESCRIPTION AND/OR PROPOSED TAXONOMIC DESIGNATION
The microorganism identified under I above was accompanied by:
☐ a scientific description
☐ a proposed taxonomic designation
(Mark with a cross where applicable)
III. RECEIPT AND ACCEPTANCE
This International Depositary Authority accepts the microorganism identified under I above,
which was received by it on September. 21. 2020 (date of the original deposit).¹
IV. RECEIPT OF REQUEST FOR CONVERSION
The microorganism identified under I above was received by this International Depositary Authority
on (date of the original deposit) and a request to convert the original deposit to a deposit under
the Budapest Treaty was received by it on (date of receipt of request for conversion).
V. INTERNATIONAL DEPOSITARY AUTHORITY
Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: September. 21. 2020.

¹ Where Rule 6.4(d) applies, such date is the date on which the status of international depositary authority was
acquired.

## Claims

1. An L-histidine producing microorganism, modified to express a protein having at least 60% sequence homology with SEQ ID NO: 12, SEQ ID NO: 13, or a combination thereof.

2. The L-histidine producing microorganism according to claim 1, wherein the protein is a foreign protein.

3. The L-histidine producing microorganism according to claim 1, wherein the modification is by introduction of a gene encoding an amino acid sequence having at least 60% sequence homology with SEQ ID NO: 12, SEQ ID NO: 13, or a combination thereof.

4. The L-histidine producing microorganism according to claim 1, wherein the protein is represented by an amino acid sequence of SEQ ID NOs: 12 and 13, SEQ ID NOs: 41 and 42, or SEQ ID NOs: 44 and 45.

5. The L-histidine producing microorganism according to claim 4, wherein the modification is by introduction of a gene encoding an amino acid sequence of SEQ ID NOs: 12 and 13, SEQ ID NOs: 41 and 42, or SEQ ID NOs: 44 and 45.

6. The L-histidine producing microorganism according to any one of claims 1 to 5, wherein the microorganism is of the genus *Corynebacterium* or the genus *Escherichia.*

7. The L-histidine producing microorganism according to claim 6, wherein the microorganism is *Corynebacterium glutamicum* or *Escherichia coli.*

8. A composition for producing L-histidine, comprising
a protein having at least 60% sequence homology with SEQ ID NO: 12, SEQ ID NO: 13, or a combination thereof,
a gene encoding the protein,
a recombinant vector comprising the gene, or
a recombinant microorganism comprising the gene or the recombinant vector.

9. The composition for producing L-histidine according to claim 8, wherein the protein is represented by an amino acid sequence of SEQ ID NOs: 12 and 13, SEQ ID NOs: 41 and 42, or SEQ ID NOs: 44 and 45.

10. The composition for producing L-histidine according to claim 8 or claim 9, wherein the microorganism is of the genus *Corynebacterium* or the genus *Escherichia.*

11. The composition for producing L-histidine according to claim 10, wherein the microorganism is *Corynebacterium glutamicum* or *Escherichia coli.*

12. A method for producing L-histidine, comprising culturing the L-histidine producing microorganism according to any one of claims 1 to 5 in a medium.

13. The method for producing L-histidine according to claim 12, further comprising recovering L-histidine from the cultured microorganism or medium, after the culturing.

14. The method for producing L-histidine according to claim 12, wherein the microorganism is of the genus *Corynebacterium* or the genus *Escherichia.*

15. The method for producing L-histidine according to claim 14, wherein the microorganism is *Corynebacterium glutamicum* or *Escherichia coli.*
